# EUROPEAN PATENT APPLICATION

(11) **EP 4 470 481 A1**
(43) Date of publication of application: **04.12.2024**
(21) Application number: 23746255.1
(22) Date of filing: 19.01.2023
(51) Int. Cl.: A61B 17/12

(54) **CONVEYING APPARATUS FOR SPRING RING**

(30) Priority: 26.01.2022 CN 202210096097
(71) Applicant: Shanghai Shenqi Medical Technology Co., Ltd., Shanghai 201203 (CN)
(72) Inventor: GU, Yingying, Shanghai 201203 (CN); ZHOU, Guolei, Shanghai 201203 (CN); WANG, Sen, Shanghai 201203 (CN); DAI, Zhihao, Shanghai 201203 (CN); XIA, Xiuli, Shanghai 201203 (CN)
(74) Representative: Ricker, Mathias
(86) International application number: PCT/CN2023/073164
(87) International publication number: WO 2023/143376

(57) **Abstract**

Provided is a delivery device for a spring coil. The delivery device for a spring coil includes a delivery unit (1), a spring coil unit (2), and an introducer sheath unit (3). A proximal end of the spring coil unit (2) is detachably connected to a distal end of the delivery unit (1). A first cone tube (32) is disposed at a distal end of an introducer sheath (3), and the diameter of the first cone tube (32) gradually decreases from a proximal end of the first cone tube (32) to a distal end of the first cone tube (32).

## Description

This application claims priority to Chinese Patent Application No. 202210096097.5 filed Jan. 26, 2022, the disclosure of which is incorporated herein by reference in its entirety.

### TECHNICAL FIELD

The present application relates to the technical field of medical devices, for example, a delivery device for a spring coil.

### BACKGROUND

As an embolic device for the treatment of aneurysms, spring coils are one of the most effective treatment methods in related technologies. After a spring coil is implanted at a target position, controlled detachment of the spring coil from the implanted position is required. Generally, the number of spring coils used in one operation is 5 to 7 on average. After several spring coils are wound and stacked, the spring coils need to be released in a light and flexible manner to prevent damage to the spring coils that have been stuffed into the tumor. Therefore, the rapid, safe, and effective detachment of spring coils plays an important role in the safety of surgery and the life safety of patients. The detachment performance of the spring coils becomes a major indicator of performance evaluation.

In the related technologies, there are four main types of detachment methods for spring coils, that is, electric detachment, gas detachment, water detachment, and mechanical detachment. The electric detachment is performed by heating the electric heating part and thus transferring heat to the core wire of polymer material in the inner cavity of spring coils to detach the spring coils. The principle of gas detachment is as follows: An external inflatable device inflates the chamber through an inflatable sealing member so that the internal elastic balloon expands and fits the internal cavity wall. Under the action of the elastic balloon, the elastic balloon shrinks and exits after spring coils are delivered to the target position. Thus, the spring coils can be detached. Water detachment is as follows: In a conveying process, a solvent is injected into a conveying tube to dissolve a fixed part. Thus, spring coils can be detached. Mechanical detachment is as follows: The components in a conveying system are connected to the proximal ends of spring coils, and then the conveying system moves the spring coils in the catheter. When the spring coils reach the target position, the conveying components in the conveying system are separated from the spring coils, and thus the spring coils are detached.

Compared with the other three methods, mechanical detachment does not require external transmission of energy, streamlines the production process of the entire spring coil conveying system, and reduces energy consumption. More importantly, mechanical detachment greatly reduces the risk of surgical failure due to the destructiveness of the device itself. Based on the preceding advantages, mechanical detachment becomes the current mainstream detachment method of spring coils.

However, mechanical detachment also has disadvantages. During the process of pushing spring coils, the friction between spring coils, a conveying component, and an introducer sheath makes it difficult to push the spring coils. In particular, a spring coil with a larger model and a larger specification is very long, and the existence of friction resistance makes it difficult to push the spring coil and may even cause the spring coil to break. In addition, certain compatibility exists between the sizes of the spring coils, the conveying component, the introducer sheath, and the guide catheter. If the inner diameter of the introducer sheath is excessively large, the spring coils will detach from the conveying component early in the process where the spring coils are conveyed to the guide catheter, making it impossible to continue pushing, and the spring coil delivery device will be scrapped. If the inner diameter of the introducer sheath is too small, the preceding excessive friction will occur, which is also undesirable.

### SUMMARY

The present application provides a delivery device for a spring coil, which can improve the adaptability of a spring coil unit and a delivery unit to a guide catheter and make it easy to push the spring coil unit and the delivery unit.

An embodiment provides a delivery device for a spring coil. The device includes a delivery unit, a spring coil unit, and an introducer sheath unit.

A proximal end of the spring coil unit is detachably connected to a distal end of the delivery unit.

The introducer sheath unit includes an introducer sheath. The spring coil unit and the delivery unit are disposed in the introducer sheath. A first cone tube is disposed at a distal end of the introducer sheath. The diameter of the first cone tube gradually decreases from a proximal end to a distal end.

In an embodiment, the diameter of the introducer sheath gradually increases from the proximal end of the introducer sheath to the distal end of the introducer sheath.

In an embodiment, the introducer sheath includes a delivery tube, a straight tube, and a second cone tube, the diameter of the straight tube is smaller than the diameter of the delivery tube, a distal end of the delivery tube is connected to a proximal end of the first cone tube, and a proximal end of the delivery tube is connected to a distal end of the straight tube through the second cone tube.

In an embodiment, the introducer sheath is provided with a limiting member configured to position the delivery unit or the spring coil unit within the introducer sheath.

In an embodiment, the delivery unit includes a push rod and a flexible connection assembly.

A proximal end of the flexible connection assembly is connected to a distal end of the push rod. A distal end of the flexible connection assembly is detachably connected to the proximal end of the spring coil unit.

As a preferred technical solution for the preceding delivery device for a spring coil, the diameter of the push rod gradually decreases from the proximal end of the push rod to the distal end of the push rod.

In an embodiment, the flexible connection assembly includes a heat shrink tube and a conveying elastic member.

One end of the heat shrink tube is sleeved on a distal end of the push rod.

A proximal end of the conveying elastic member is connected to the other end of the heat shrink tube. The spring coil unit is detachably connected to a distal end of the conveying elastic member.

As a preferred technical solution for the preceding delivery device for a spring coil, the conveying elastic member includes a conveying spring and a developing spring.

A proximal end of the conveying spring is connected to the heat shrink tube.

A proximal end of the developing spring is connected to a distal end of the conveying spring. The developing spring is coated with developer.

In an embodiment, the spring coil unit includes a spring coil, a first interlocking member is connected to a proximal end of the spring coil, and the distal end of the delivery unit is provided with a second interlocking member configured to engage with or separate from the first interlocking member.

In an embodiment, the limiting member is a locking thread disposed on the introducer sheath.

### BRIEF DESCRIPTION OF DRAWINGS

FIG. 1 is a diagram illustrating the partial structure of a delivery device for a spring coil according to an embodiment of the present application.
FIG. 2 is a diagram illustrating the structure of an introducer sheath unit according to an embodiment of the present application.
FIG. 3 is a diagram illustrating part of the connection between a straight tube and a second cone tube according to an embodiment of the present application.
FIG. 4 is a diagram illustrating the structure of a delivery unit according to an embodiment of the present application.
FIG. 5 is a diagram illustrating the structure of the connection between a second interlocking member and a developing spring according to an embodiment of the present application.

### Reference list

- 1: delivery unit
- 2: spring coil unit
- 3: introducer sheath unit
- 11: push rod
- 12: flexible connection assembly
- 121: heat shrink tube
- 122: conveying spring
- 123: developing spring
- 13: second interlocking member
- 21: spring coil
- 22: first interlocking member
- 23: guard
- 31: introducer sheath
- 311: delivery tube
- 312: straight tube
- 313: second cone tube
- 32: first cone tube
- 33: limiting member

### DETAILED DESCRIPTION

In the description of the present application, unless otherwise expressly specified and limited, a term "connected to each other", "connected", or "secured" is to be construed in a broad sense, for example, as securely connected, detachably connected, or integrated; mechanically connected or electrically connected; directly connected to each other or indirectly connected to each other via an intermediary; or internally connected between two components or interaction relations between two components. For those of ordinary skill in the art, specific meanings of the preceding terms in the present application may be construed according to specific situations.

In the present application, unless otherwise expressly specified and limited, when a first feature is described as "above" or "below" a second feature, the first feature and the second feature may be in direct contact or be in contact via another feature between the two features instead of being in direct contact. Moreover, when the first feature is "on", "above", or "over" the second feature, the first feature is right on, above, or over the second feature, or the first feature is obliquely on, above, or over the second feature, or the first feature is simply at a higher level than the second feature. When the first feature is "under", "below", or "underneath" the second feature, the first feature is right under, below, or underneath the second feature, or the first feature is obliquely under, below, or underneath the second feature, or the first feature is simply at a lower level than the second feature.

In the related technology, if the inner diameter of an introducer sheath is excessively large, a spring coil detaches from a conveying component early in the process where the spring coil is conveyed to a guide catheter, making it impossible to continue pushing, and the delivery device for a spring coil is scrapped; if the inner diameter of the introducer sheath is too small, the friction between the spring coil, the conveying component, and the introducer sheath is great, which makes it difficult to push the spring coil and may even cause the spring coil to break. Therefore, it is urgently necessary to provide a delivery device for a spring coil to resolve the preceding technical problems.

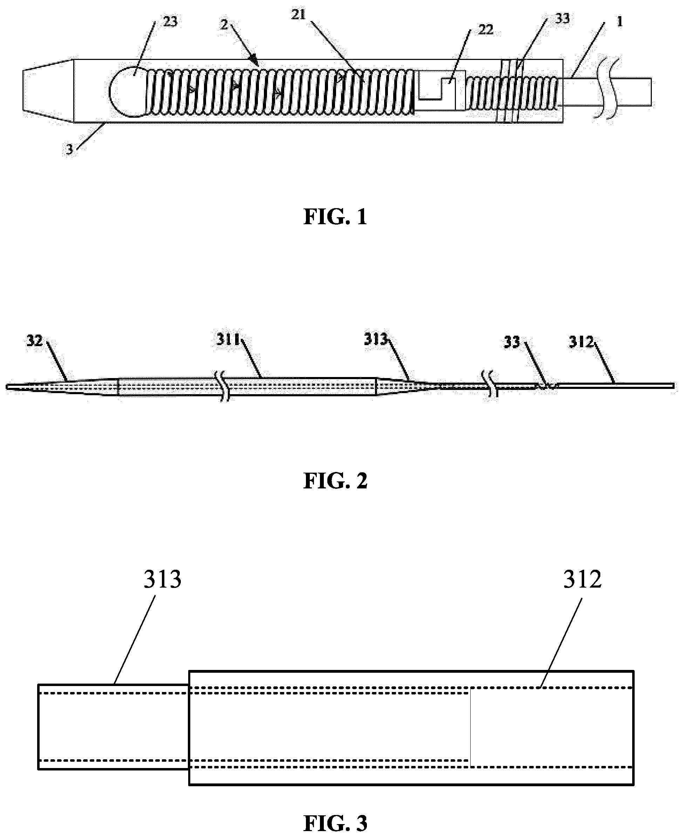

As shown in FIG. 1 and FIG. 2, the delivery device for a spring coil provided in this embodiment includes a delivery unit 1, a spring coil unit 2, and an introducer sheath unit 3; the delivery unit 1 pushes the spring coil unit 2 to move in the introducer sheath unit 3 and enters a guide catheter through the introducer sheath unit 3. After the guide catheter directs the spring coil unit 2 to the target position, the spring coil unit 2 is separated from the delivery unit 1, and the spring coil unit 2 is implanted into the target position.

In an embodiment, a proximal end of the spring coil unit 2 is detachably connected to a distal end of the delivery unit 1; the introducer sheath unit 3 includes an introducer sheath 31, the spring coil unit 2 and the delivery unit 1 are disposed in the introducer sheath 31, a first cone tube 32 is disposed at a distal end of the introducer sheath 31, and the diameter of the first cone tube 32 gradually decreases from a proximal end of the first cone tube 32 to a distal end of the first cone tube 32. A small end of the first cone tube 32 is convenient for connecting to the guide catheter. A large end of the first cone tube 32 is connected to the introducer sheath 31. The diameter of the introducer sheath 31 may be set slightly larger to reduce the friction between the spring coil unit 2 and the introducer sheath 31, thereby making the spring coil unit 2 less likely to deform or break during the pushing process. The first cone tube 32 is disposed at a distal end of the introducer sheath 31 so that the adaptation between the introducer sheath 31 and the guide catheter is improved. Moreover, it is ensured that the delivery unit 1 is effectively connected to the spring coil unit 2 in the process of entering the guide catheter, so as to avoid the problems that the delivery unit 1 and the spring coil unit 2 are separated in advance and cannot be pushed and that the delivery device for a spring coil is scrapped.

In this embodiment, the diameter of the introducer sheath 31 gradually increases from the proximal end of the introducer sheath 31 to the distal end of the introducer sheath 31, and the portion with a small diameter may limit the movement of the delivery unit 1 and the spring coil unit 2 to a certain extent to avoid unnecessary movement of the delivery unit 1 and the spring coil unit 2 in the introducer sheath 31 which causes the delivery device for a spring coil to fail. When the spring coil unit 2 is pushed, after the delivery unit 1 and the spring coil unit 2 enter a portion with a large diameter of the introducer sheath 31, the friction between the spring coil unit 2 and the introducer sheath 31 is reduced in the conveying process, thereby making the spring coil unit 2 less likely to deform or break during the pushing process. The proximal end in this embodiment refers to the direction above in FIG. 2 and the direction to the right in FIG. 1. The distal end refers to the direction below in FIG. 2 and the direction to the left in FIG. 1.

In an embodiment, the introducer sheath 31 includes a delivery tube 311 and a straight tube 312, and the diameter of the straight tube 312 is smaller than the diameter of the delivery tube 311. When the delivery device for a spring coil is not used, the delivery unit 1 and the spring coil unit 2 are disposed in the straight tube 312 to limit the displacement of the delivery unit 1 and the spring coil unit 2. When the spring coil unit 2 is pushed, the spring coil unit 2 and the delivery unit 1 gradually enter the delivery tube 311 with a large diameter, thus reducing the friction between the spring coil unit 2 and the introducer sheath 31 in the conveying process. To facilitate the connection between the straight tube 312 and the delivery tube 311, a proximal end of the delivery tube 311 in this embodiment is connected to a distal end of the straight tube 312 through a second cone tube 313. A distal end of the delivery tube 311 is connected to a proximal end of the first cone tube 32.

The straight tube 312, the delivery tube 311, the first cone tube 32, and the second cone tube 313 are usually made of polymer materials, for example, polyolefin materials such as polyethylene and polypropylene. Therefore, an end of the second cone tube 313 is inserted into the straight tube 312 (as shown in FIG. 3) and connects the straight tube 312 to the second cone tube 313 by hot melting. The connection method is simple, and the structural strength is good.

To ensure that the spring coil unit 2 and the delivery unit 1 are not displaced in the introducer sheath 31 when the delivery device for a spring coil is not used, the introducer sheath 31 in this embodiment may be provided with a limiting member 33 configured to position the delivery unit 1 or the spring coil unit 2 within the introducer sheath 31. When the delivery device for a spring coil is used, the limitation of the displacement of the spring coil unit 2 and the delivery unit 1 by the limiting member 33 may be released. In an embodiment, the limiting member 33 is disposed in the straight tube 312 of the introducer sheath 31.

Since the straight tube 312 is made of polymer material and has a certain degree of flexibility, the limiting member 33 includes a locking thread disposed on the straight tube 312. That is, after the delivery device for a spring coil is assembled, part of the straight tube 312 is screwed so that part of the straight tube 312 is deformed, the inner diameter becomes smaller, and the inner wall of the straight tube 312 abuts against the delivery unit 1, thereby preventing the delivery unit 1 and the spring coil unit 2 from moving. When the delivery device for a spring coil is used, the straight tube 312 is screwed reversely so that the inner wall of the straight tube 312 is away from the delivery unit 1 to achieve unlocking. In other embodiments, the limiting member 33 may also be a latch, a latch hole is disposed on the straight tube 312, and the latch passes through the latch hole and abuts against the delivery unit 1. When the delivery device for a spring coil is used, the latch is moved outward to separate the latch from the delivery unit 1.

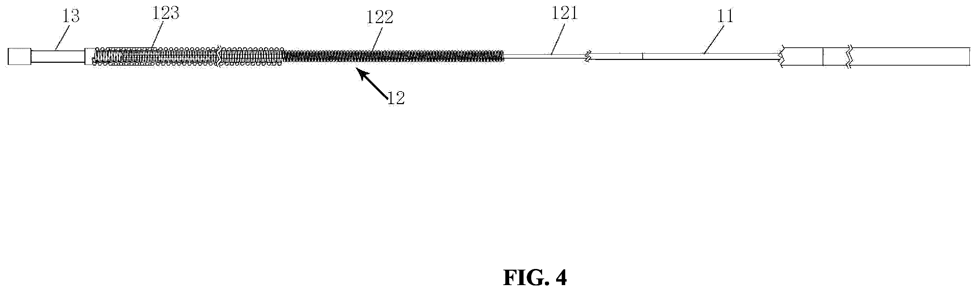

The delivery unit 1 is configured to push the spring coil unit 2 to move within the introducer sheath 31. In an embodiment, as shown in FIG. 4, the delivery unit 1 in this embodiment includes a push rod 11, a distal end of the push rod 11 is connected to a flexible connection assembly 12, and a distal end of the flexible connection assembly 12 is detachably connected to the proximal end of the spring coil unit 2. The delivery unit 1 is designed with a segmented structure and has flexible connection portions. This configuration has good adaptability and good passability in relatively tortuous parts of peripheral blood vessels, which improves the conveying performance of the delivery device for a spring coil.

The diameter of the push rod 11 gradually decreases from the proximal end of the push rod 11 to the distal end of the push rod 11. The diameter at the proximal end of the push rod 11 is large to provide a certain supporting force when the spring coil unit 2 is pushed. The rod diameter gradually decreases toward the distal end to facilitate the movement of the push rod 11 in the introducer sheath 31, thereby reducing the friction between the push rod 11 and the introducer sheath 31.

In this embodiment, the length of the push rod 11 may be set to 600 mm to 2000 mm, and the rod diameter of the push rod 11 is in the range of 0.5 mm to 2 mm. Certainly, the length and the rod diameter of the push rod 11 are not limited to the preceding value ranges and may be set as required. The push rod 11 is made of biocompatible metal or alloy. In an embodiment, the push rod 11 is made of stainless steel or nickel-titanium material. The push rod 11 has a certain flexibility, is not easy to deform, and has a strong recovery capability so that it is convenient for the delivery device for a spring coil to deliver the spring coil unit 2 in circuitous blood vessel positions.

The preceding flexible connection assembly 12 includes a heat shrink tube 121 and a conveying elastic member. A first end of the heat shrink tube 121 is sleeved on a distal end of the push rod 11. A second end of the heat shrink tube 121 is connected to a proximal end of the conveying elastic member. The spring coil unit 2 is detachably connected to a distal end of the conveying elastic member. The heat shrink tube 121 implements the flexible connection between the conveying elastic member and the push rod 11. The heat shrink tube 121 is usually made of olefin materials such as polypropylene and polyethylene. Therefore, by locally heating the heat shrink tube 121 at a suitable temperature, the heat shrink tube 121 is deformed to be fixedly connected to the push rod 11 and the conveying elastic member. For a relatively precise component, the connection is relatively convenient, the operation is easy, and costs are low.

The inner diameter of the heat shrink tube 121 matches the rod diameter of the push rod 11 and the size of the spring coil unit 2. In this embodiment, the inner diameter of the heat shrink tube 121 is generally 0.8 mm to 2.5 mm. To not affect the conveying capability of the delivery unit 1 and to adapt well to tortuous blood vessel positions and ensure that the delivery unit 1 has a certain structural strength, the heat shrink tube 121 in this embodiment has a wall thickness of 0.2 mm to 0.8 mm.

To further improve the bending and deformation capability of the delivery unit 1, the conveying elastic member in this embodiment includes a conveying spring 122 and a developing spring 123, a proximal end of the conveying spring 122 is connected to the heat shrink tube 121, and a proximal end of the developing spring 123 is connected to a distal end of the conveying spring 122, thereby further improving the ability of the delivery unit 1 to adapt to tortuous blood vessel positions. The conveying spring 122 and the developing spring 123 are connected by welding, and the two are generally tubular structures. In this embodiment, the outer diameter of the conveying spring 122 is 1 mm to 2 mm, and the length of the conveying spring 122 is 8 mm to 20 mm. In addition, the conveying spring 122 is usually made of biocompatible metal or alloy, specifically made of a material such as stainless steel or nickel-titanium. The conveying spring 122 has a certain flexibility, is not easy to deform, and has a strong recovery capability so that it is convenient for the delivery device for a spring coil to deliver the spring coil unit 2 in circuitous blood vessel positions.

The developing spring 123 is coated with developer to facilitate the positioning of the spring coil unit 2 when the delivery device for a spring coil is used. In this embodiment, the developing spring 123 is made of biocompatible metal or alloy, specifically made of platinum-tungsten alloy.

In this embodiment, the delivery unit 1 is arranged in sections so that the adaptability of the delivery device for a spring coil in circuitous blood vessel positions is improved, and good passability is provided.

In the conveying process, the spring coil unit 2 is detached from the delivery unit 1. After the spring coil unit 2 is directed to the target position, the spring coil unit 2 is separated from the delivery unit 1, and the spring coil unit 2 is implanted into the target position. In this embodiment, with continued reference to FIG. 1 and FIG. 4, the spring coil unit 2 includes a spring coil 21, a first interlocking member 22 is connected to a proximal end of the spring coil 21, and the distal end of the delivery unit 1 is provided with a second interlocking member 13 configured to engage with or separate from the first interlocking member 22.

In an embodiment, the second interlocking member 13 is connected to a distal end of the developing spring 123 of the delivery unit 1, the second interlocking member 13 and the first interlocking member 22 include at least one interlocking notch, an interlocking protrusion of the second interlocking member 13 adjacent to an interlocking notch is inserted into the notch of the first interlocking member 22, and an interlocking protrusion of the first interlocking member 22 adjacent to an interlocking notch is inserted into the interlocking notch of the second interlocking member 13. In this manner, the spring coil unit 2 engages with and is connected to the delivery unit 1, and the structure is simple.

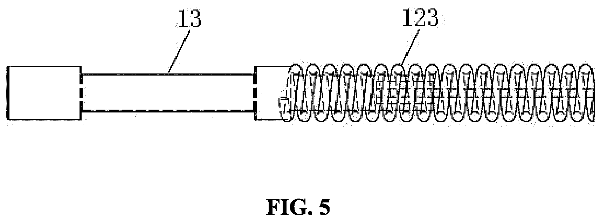

With reference to FIG. 5, the second interlocking member 13 includes a support rod. The support rod is inserted into the distal end of the developing spring 123. The support rod and the developing spring 123 are fixedly connected by welding. The connection structure has high strength. The second interlocking member 13 is made of biocompatible metal or alloy. In an embodiment, the material of the second interlocking member 13 may be the same as the material of the development spring 123 to be fixedly connected to the development spring 123.

With continued reference to FIG. 1, a fiber bundle is disposed on the spring coil 21 that needs to be delivered to the target tissue in this embodiment. In this manner, when the spring coil 21 is released, the friction force in the introducer sheath 31 and the guide catheter is smaller, and the pushing force can be transmitted more easily and better in the axial direction of the spring coil 21 without the stacking of the spring coil 21, thus reducing the difficulty of the surgical operation and the risk of aneurysm rupture. The first interlocking member 22 is fixedly connected to the spring coil 21 by welding or riveting. The spring coil 21 and the first interlocking member 22 are made of biocompatible metal or alloy. In an embodiment, the material of the spring coil 21 and the first interlocking member 22 may be stainless steel, titanium alloy, or platinum alloy.

In this embodiment, an end guard 23 is disposed at the distal end of the spring coil 21 to avoid destroying the aneurysm when the spring coil 21 is released.

The delivery device for a spring coil provided in this embodiment adopts a mechanical method to release the spring coil 21 without using external energy input. In this manner, the production process and operating method of the delivery device for a spring coil are simplified, and the safety of using the delivery device for a spring coil is improved. The embodiment optimizes the delivery device for a spring coil in terms of many aspects such as the production process, security, and product effectiveness. The optimized device has a simple structure, is easy to operate, and features high safety.

## Claims

1. A delivery device for a spring coil, comprising:
a delivery unit (1);
a spring coil unit (2), wherein a proximal end of the spring coil unit (2) is detachably connected to a distal end of the delivery unit (1); and
an introducer sheath unit (3), comprising an introducer sheath (31), wherein the spring coil unit (2) and the delivery unit (1) are disposed in the introducer sheath (31), a first cone tube (32) is disposed at a distal end of the introducer sheath (31), and a diameter of the first cone tube (32) gradually decreases from a proximal end of the first cone tube (32) to a distal end of the first cone tube (32).

2. The delivery device for the spring coil of claim 1, wherein a diameter of the introducer sheath (31) gradually increases from a proximal end of the introducer sheath (31) to the distal end of the introducer sheath (31).

3. The delivery device for the spring coil of claim 2, wherein the introducer sheath (31) comprises a delivery tube (311), a straight tube (312), and a second cone tube (313), a diameter of the straight tube (312) is smaller than a diameter of the delivery tube (311), a distal end of the delivery tube (311) is connected to the proximal end of the first cone tube (32), and a proximal end of the delivery tube (311) is connected to a distal end of the straight tube (312) through the second cone tube (313).

4. The delivery device for the spring coil of claim 1, wherein the introducer sheath (31) is provided with a limiting member (33) configured to position the delivery unit (1) or the spring coil unit (2) within the introducer sheath (31).

5. The delivery device for the spring coil of claim 1, wherein the delivery unit (1) comprises:
a push rod (11); and
a flexible connection assembly (12), wherein a proximal end of the flexible connection assembly (12) is connected to a distal end of the push rod (11), and a distal end of the flexible connection assembly (12) is detachably connected to the proximal end of the spring coil unit (2).

6. The delivery device for the spring coil of claim 5, wherein a diameter of the push rod (11) gradually decreases from a proximal end of the push rod (11) to the distal end of the push rod (11).

7. The delivery device for the spring coil of claim 5, wherein the flexible connection assembly (12) comprises:
a heat shrink tube (121), wherein a first end of the heat shrink tube (121) is sleeved on the distal end of the push rod (11); and
a conveying elastic member, wherein a proximal end of the conveying elastic member is connected to a second end of the heat shrink tube (121), and the spring coil unit (2) is detachably connected to a distal end of the conveying elastic member.

8. The delivery device for the spring coil of claim 7, wherein the conveying elastic member comprises:
a conveying spring (122), wherein a proximal end of the conveying spring (122) is connected to the heat shrink tube (121); and
a developing spring (123), wherein a proximal end of the developing spring (123) is connected to a distal end of the conveying spring (122), and the developing spring (123) is coated with developer.

9. The delivery device for the spring coil of claim 1, wherein the spring coil unit (2) comprises a spring coil (21), a first interlocking member (22) is connected to a proximal end of the spring coil (21), and the distal end of the delivery unit (1) is provided with a second interlocking member (13) configured to engage with or separate from the first interlocking member (22).

10. The delivery device for the spring coil of claim 4, wherein the limiting member (33) is a locking thread disposed on the introducer sheath (31).
